# EUROPEAN PATENT SPECIFICATION

(11) **EP 3 285 756 B1**
(45) Date of publication and mention of the grant of the patent: **22.02.2023**
(21) Application number: 16783692.3
(22) Date of filing: 19.04.2016
(51) Int. Cl.: A61K 31/195, A61K 31/192, A61P 1/16, A61K 9/08

(54) **FORMULATIONS OF L-ORNITHINE PHENYLACETATE**
FORMULIERUNGEN VON L-ORNITHIN-PHENYLACETAT
FORMULATIONS DE PHÉNYLACÉTATE DE L-ORNITHINE

(30) Priority: 20.04.2015 US 201562150238 P; 21.04.2015 US 201562150676 P; 28.08.2015 US 201562211619 P; 13.11.2015 US 201562255300 P; 08.01.2016 US 201662276754 P
(43) Date of publication of application: 28.02.2018
(73) Proprietor: Ocera Therapeutics, Inc., Redwood City, CA 94065 (US)
(72) Inventor: RUETTIMANN, Kenneth, Raleigh, NC 27612 (US); COFFIN, Mark, Durham, NC 27703 (US); BERNSTEIN, James, Raleigh, NC 27615 (US); GOODSON, Gary, Raleigh, NC 27615 (US); WANG, Laurene, Chapel Hill, NC 27516 (US)
(74) Representative: Jones, Nicholas Andrew
(86) International application number: PCT/US2016/028298
(87) International publication number: WO 2016/172112

(56) References cited:
- WO-A1-2006/056794
- WO-A1-2006/056794
- WO-A1-2010/115055
- WO-A1-2012/048043
- WO-A2-2010/144498
- WO-A2-2010/144498
- AU-A1- 2014 250 643
- AU-A1- 2015 221 466
- VENTURA-COTS MERITXELL ET AL: "Safety of Ornithine Phenylacetate in Cirrhotic Decompensated Patients : An Open-label, Dose-escalating, Single-cohort Study", JOURNAL OF CLINICAL GASTROENTEROLOGY, RAVEN PRESS LTD., NEW YORK, NY, US , vol. 47, no. 10 1 January 2013 (2013-01-01), pages 881-887, XP009507196, ISSN: 0192-0790, DOI: 10.1097/MCG.0B013E318299C789 Retrieved from the Internet: URL:http://content.wkhealth.com/linkback/o penurl?sid=WKPTLP:landingpage&an=00004836- 201311000-00013
- JALAN R ET AL: "l-Ornithine phenylacetate (OP): A novel treatment for hyperammonemia and hepatic encephalopathy", MEDICAL HYPOTHESES, EDEN PRESS, PENRITH, US, vol. 69, no. 5, 27 April 2007 (2007-04-27) , pages 1064-1069, XP029096478, ISSN: 0306-9877, DOI: 10.1016/J.MEHY.2006.12.061
- YTREBO LARS MARIUS ET AL: "L-ornithine phenylacetate attenuates increased arterial and extracellular brain ammonia and prevents intracranial hypertension in pigs with acute liver failure", HEPATOLOGY, JOHN WILEY & SONS, INC, US, vol. 50, no. 1, 1 July 2009 (2009-07-01), pages 165-174, XP009164051, ISSN: 0270-9139, DOI: 10.1002/HEP.22917 [retrieved on 2009-02-18]
- Rune Gangsøy Kristiansen ET AL: "L-Ornithine phenylacetate reduces ammonia in pigs with acute liver failure through phenylacetylglycine formation: a novel ammonia-lowering pathway", American journal of physiology. Gastrointestinal and liver physiology, 15 November 2014 (2014-11-15), pages G1024-G1031, XP055532599, United States DOI: 10.1152/ajpgi.00244.2014 Retrieved from the Internet: URL:https://www.physiology.org/doi/full/10 .1152/ajpgi.00244.2014
- VAIRAPPAN BALASUBRAMANIYAN ET AL: "Ammonia reduction with ornithine phenylacetate restores brain eNOS activity via the DDAH-ADMA pathway in bile duct-ligated cirrhotic rats", AMERICAN JOURNAL OF PHYSIOLOGY - GASTROINTESTINAL AND LIVER PHYSIOLOGY, vol. 302, no. 1, 1 January 2012 (2012-01-01), pages G145-G152, XP055532625, US ISSN: 0193-1857, DOI: 10.1152/ajpgi.00097.2011
- VENTURA-COTS, M. ET AL.: 'Safety of ornithine phenylacetate in cirrhotic decompensated patients: an open-label, dose-escalating, single-cohort study' JOURNAL OF CLINICAL GASTROENTEROLOGY. vol. 47, no. 10, 2013, pages 881 - 887, XP009507196
- JALAN, R. ET AL.: 'L-Ornithine phenylacetate (OP): a novel treatment for hyperammonemia and hepatic encephalopathy' MEDICAL HYPOTHESES. vol. 69, no. 5, 2007, pages 1064 - 1069, XP029096478
- JOVER-COBOS, M. ET AL.: 'Ornithine phenylacetate revisited' METABOLIC BRAIN DISEASE. vol. 28, no. 2, 2013, pages 327 - 331, XP055324717
- M. MOKHTARANI ET AL: "Urinary phenylacetylglutamine as dosing biomarker for patients with urea cycle disorders", MOLECULAR GENETICS AND METABOLISM, vol. 107, no. 3, 1 November 2012 (2012-11-01), pages 308-314, XP055202395, ISSN: 1096-7192, DOI: 10.1016/j.ymgme.2012.08.006

## Description

### BACKGROUND

The present application relates to pharmaceutical compositions comprising oral formulations of L-ornithine phenylacetate and the use for treating hyperammonemia in patients having various acute and chronic liver diseases and disorders, for example, acute liver failure, liver cirrhosis, liver decompensation, portal hypertension, hepatic encephalopathy, or patients with urea cycle disorders.

Chronic liver disease is characterized by the gradual destruction of liver tissue over time, whereby healthy and regenerating liver tissue is slowly replaced with scar and necrotic tissue. This is known as liver cirrhosis. Normal liver function is impaired and the scar tissue progressively diminishes blood flow through the liver. As normal regenerating liver tissue is lost, nutrients, hormones, drugs and toxins are no longer effectively processed. This can result in symptoms including abnormal clearance of proteins absorbed through the intestinal tract, leading to accumulation of ammonia; abnormal excretion, leading to an accumulation of bilirubin in the blood, producing jaundice; increased sinusoidal pressure, leading to fluid accumulation in the abdomen (ascites); and portal hypertension (and portosystemic shunting) wherein scarred liver tissue acts as a barrier to blood flow, leading to increased portal blood pressure and oesophageal varices.

Patients with chronic liver disease can be in a fairly stable clinical state and exhibit few or no symptoms. However, such patients are at risk of an abrupt deterioration in their condition which can lead to acute-on-chronic liver failure. This transition from a "compensated" state, where the liver is able to function, albeit at a reduced level, to a "decompensated" state, where liver function fails, involves the effect of precipitating events. Precipitating events associated with chronic liver disease include gastrointestinal bleeding, infection (sepsis), portal vein thrombosis and dehydration.

Hepatic encephalopathy (HE) is a common complication of decompensated cirrhosis; it has a significant negative effect on survival even after liver transplantation and is associated with irreversible impairment in cognitive function. An estimated 60-70% of cirrhotic subjects have at least subtle signs of neurocognitive impairment, and HE is the principal diagnosis in hospitalized subjects. Overt HE has a prevalence of approximately 30% in the cirrhotic population, and accounts for about 150,000 hospitalizations annually in the United States.

Hepatic encephalopathy (HE) is a complex neuropsychiatric disorder that occurs in diverse clinical situations such as acute or chronic liver disease and spontaneous portosystemic venous shunting. In the early stages of hepatic encephalopathy subtle mental changes occur such as poor concentration, confusion and disorientation. In severe cases, hepatic encephalopathy can lead to stupor, coma, brain swelling (cerebral edema) and death. In the case of patients who develop HE as a result of chronic liver disease, the onset of HE is often the result of a clinically precipitating event such as gastrointestinal bleeding, sepsis (infection), portal vein thrombosis or dehydration.

Gastrointestinal bleeding and portosystemic shunting allows toxic substances, which are usually metabolized by the liver, to bypass the liver, enter the systemic circulation and cross the blood-brain barrier to exert direct or indirect neurotoxic effects on the central nervous system. Ammonia accumulation is thought to play an important role in the progression of hepatic encephalopathy and multiorgan failure (respiratory failure, cardiovascular failure, kidney failure). In addition to ammonia, septicaemia (or bacterial peritonitis) which develops soon after a gastrointestinal bleed is also likely to be a contributing factor to hepatic encephalopathy.

Liver decompensation can then lead to multi-organ failure and hepatic encephalopathy. In the early stages of hepatic encephalopathy subtle mental changes such as poor concentration or the inability to construct simple objects occurs. In severe cases, hepatic encephalopathy can lead to stupor, coma, brain swelling and death.

Urea cycle disorder or urea cycle defect is a genetic disorder caused by a deficiency of one of the enzymes in the urea cycle which is responsible for removing ammonia from the blood stream. Normally, the urea is transferred into the urine and removed from the body. In urea cycle disorders, the nitrogen accumulates in the form of ammonia, a toxic substance, and is not removed from the body. It has been reported that sodium phenylbutyrate may be used in the management of this condition. *See,* for example, Batshaw, M. L. et al., "Alternative pathway therapy for urea cycle disorders: twenty years later," J. Pediatr. (2001) 138 (1 Suppl): S46-S55.

A common therapy for patients with hepatic encephalopathy involves strategies to reduce the concentration of ammonia. These include restriction of dietary protein intake; administration of lactulose, neomycin, L-ornithine L-aspartate (LOLA), or sodium benzoate; and cleansing enemas. There are currently marketed products that contain phenylacetic acid (e.g., AMMONUL^{®}) or prodrugs of phenylacetic acid, e.g., phenylbutyrate (BUPHENYL^{®}) or glycerol phenylbutyrate (RAVICTI^{®}) as the ammonia scavenger (binding agent) for the treatment of hyperammonemia due to urea cycle disorder (UCDs). RAVICTI^{®} has also been evaluated in clinical trials and shown preliminary efficacy for the treatment of hepatic encephalopathy. *See,* for example, Rockey D. et al., "Randomized, Double-Blind, Controlled Study of Glycerol Phenylbutyrate in Hepatic Encephalopathy," Hepatology, 2014, 59(3):1073-1083. In addition, L-ornithine phenylacetate has been reported to be an effective treatment of hyperammonemia and hepatic encephalopathy. Jalan *et al.,* reported a clinical study where the data showed that L-ornithine phenylacetate is useful in ammonia lowering. *See* Jalan et al., "L-Ornithine phenylacetate (OP): a novel treatment for hyperammonemia and hepatic encephalopathy," Med Hypotheses 2007; 69(5):1064-69. *See also,* U.S. Publication Nos. 2008/0119554, 2010/0280119, and 2013/0211135.

Currently, L-ornithine phenylacetate is under a double-blind placebo-controlled Phase 2b clinical trial for the treatment of acute hepatic encephalopathy. The Interim analysis suggested a promising treatment effect with L-ornithine phenylacetate and a reduction in time to recovery for patients with acute hepatic encephalopathy. *See* April 1, 2015 Ocera Therapeutics, Inc. Press Release.

Typically, L-ornithine phenylacetate has excellent solubility in water or aqueous solution. In all the known clinical studies of L-ornithine phenylacetate for treating acute or chronic liver diseases, L-ornithine phenylacetate is administered by intravenous infusion over a period of time, for example, from 1 day or up to five days in human studies. There exists a need to develop alternative administration routes to improve patient convenience. WO2010144498 discloses the use of L-ornithine in combination with at least one of phenylacetate and phenylbutyrate to treat chronic liver disease with hyperammonaemia by intraperitoneal administration. WO2006056794 discloses the use of L-ornithine in combination with at least one of phenylacetate and phenylbutyrate to treat chronic liver disease with hyperammonaemia. Ornithine is administered intravenously.

### SUMMARY

The section headings used herein are for organizational purposes only and are not to be construed as limiting the subject matter described.

The present application relates to oral pharmaceutical formulations, comprising L-ornithine phenylacetate at an oral dosage of about 2.0 g to about 9.0 g, and one or more pharmaceutically acceptable excipients or carriers, wherein the formulation is suitable for administration as a single oral dose and provides an immediate release profile of L-ornithine phenylacetate upon oral administration. In some embodiments, the oral dosage of L-ornithine phenylacetate is from about 5.0 g to about 8.0 g.

Also described herein are oral pharmaceutical formulations comprising an effective amount of L-ornithine phenylacetate, and one or more pharmaceutically acceptable excipients or carriers, wherein upon oral administration, the formulation has a controlled release profile of L-ornithine and phenylacetate. Also described herein are oral pharmaceutical formulations comprising a core and one or more controlled release coating layers on the core, where the core comprises L-ornithine phenylacetate and the controlled release coating layers comprise one or more polymers.

The oral pharmaceutical formulation of the present invention is useful for the treatment of hyperammonemia, said treatment comprising administering to a subject in need thereof the oral pharmaceutical formulation comprising L-ornithine phenylacetate. The oral pharmaceutical formulation of L-ornithine phenylacetate according to the invention provides an immediate release of L-ornithine phenylacetate after administration. In some embodiments, the subject has acute liver failure or chronic liver diseases. In some embodiments, the subject has liver cirrhosis or liver decompensation. In some embodiments, the subject has hepatic encephalopathy. In still some embodiments, the subject has portal hypertension.

### BRIEF DESCRIPTION OF THE DRAWINGS

**FIGs. 1A** and **1B** are microscopic images of a particle of a controlled-release oral pharmaceutical formulation (not according to the invention) of L-ornithine phenylacetate with smooth, spherical and consistent coating produced by the extrusion spheronization process.
**FIG. 2** is a line chart depicting the *in vitro* dissolution profile of phenylacetate for various coated extrudates.
**FIG. 3** is a bar chart depicting the *in vivo* pharmacokinetic profiles of L-ornithine (ORN), phenylacetate metabolites phenylacetic acid (PAA) and phenylacetylglutamine (PAGN) after oral administration of controlled-release L-ornithine phenylacetate of Formulations 3, 5 and 6 (not according to the invention) and an immediate-release L-ornithine phenylacetate solution.
**FIG. 4** is a line chart depicting the *in vivo* pharmacokinetic profiles of phenylacetic acid (PAA) in beagle dogs after oral administration of controlled-release L-ornithine phenylacetate of Formulations 3, 5 and 6 (not according to the invention) and an immediate-release L-ornithine phenylacetate solution.
**FIG. 5** is a line chart depicting the *in vivo* surrogate pharmacodynamic profiles of phenylacetylglutamine (PAGN) in beagle dogs after oral administration of controlled-release L-ornithine phenylacetate of Formulations 3, 5 and 6 (not according to the invention) and an immediate-release L-ornithine phenylacetate solution.
**FIG. 6A** is a line chart depicting the comparison of *in vitro* dissolution profile of Formulation D with Formulation A using USP Apparatus 2 Paddle Method.
**FIG. 6B** is a line chart depicting the comparison of *in vitro* dissolution profile of Formulation E with Formulation C using USP Apparatus 2 Paddle Method
**FIGs. 7A** and **7B** are Scanning Electron Micrographs (SEM) images of the small pellets of Formulation D. **FIGs. 7C** and **7D** are Scanning Electron Micrographs (SEM) images of the small pellets of Formulation E.
**FIG. 8** is a line chart depicting the *in vivo* plasma pharmacokinetic profiles of phenylacetic acid (PAA) in humans after administration of controlled-release Formulations A, B, and C, and RAVICTI^{®} (not according to the invention), and an immediate-release oral formulation of L-ornithine phenylacetate.
**FIG. 9** is a line chart depicting the *in vivo* surrogate plasma pharmacodynamic profiles of phenylacetylglutamine (PAGN) in humans after administration of controlled-release Formulations A, B, and C, and RAVICTI^{®} (not according to the invention), and an immediate-release oral formulation of L-ornithine phenylacetate.

### DETAILED DESCRIPTION OF THE EMBODIMENTS

Some embodiments of the present application are directed to oral formulations of L-ornithine phenylacetate as defined in claim 1. The invention provides a low dose formulation, using much lower doses of equivalent phenylacetate as compared to RAVICTI^{®}. The invention relates to an immediate release formulation.

### Low Dose Formulations

The present application relates to pharmaceutical formulations, comprising L-ornithine phenylacetate at a dosage of about 2.0 g to about 9.0 g, and one or more pharmaceutically acceptable excipients or carriers. The formulation provides an immediate release profile of L-ornithine phenylacetate upon administration (for example, an immediate-release oral formulation in the form of a liquid solution). The L-ornithine phenylacetate is in a dosage of about 2.0 g, about 2.5 g, about 3.0 g, about 3.5 g, about 4.0 g, about 4.5 g, about 5.0 g, about 5.5 g, about 6.0 g, about 6.5 g, about 7.0 g, about 7.5 g, about 8.0 g, about 8.5 g, or about 9.0 g, or in a dosage range defined by any of the two preceding values (for example, 5.0 g to 8.0 g). The pharmaceutical formulation is suitable for administration as a single unit dosage form. In some embodiments, the unit dosage form is a tablet, a capsule, a pill, pellets, free-flowing powder, or liquid. In some embodiments, the formulation provides conversion of phenylacetate to phenylacetylglutamine over 24 hours of greater than about 30%, greater than about 40%, greater than about 50%, greater than about 60%, greater than about 70%, greater than about 80%, or greater than about 90%. In some further embodiments, the formulation provides conversion of phenylacetate to phenylacetylglutamine over 24 hours of greater than about 80%.

The low dose pharmaceutical formulations described herein are for administration by the oral route. The pharmaceutical formulation of L-ornithine is an oral dosage form.

### Methods of Treatment

The oral pharmaceutical formulation of claim 1 is useful for treating hyperammonemia, said treatment comprising orally administering to a subject in need thereof the pharmaceutical formulation comprising an effective amount of L-ornithine phenylacetate. In some embodiments, the subject has acute liver failure or chronic liver diseases. In some embodiments, the subject has liver cirrhosis or liver decompensation. In some embodiments, the subject has hepatic encephalopathy. In still some embodiments, the subject has portal hypertension. In some embodiments, the subject has a urea cycle disorder.

L-ornithine phenylacetate may be administered in a dose ranging from about 10 mg/kg to about 1000 mg/kg, from about 20 mg/kg to about 900 mg/kg, from about 30 mg/kg to about 800 mg/kg, from about 40 mg/kg to about 700 mg/kg, or from about 50 mg/kg to about 600 mg/kg. L-ornithine phenylacetate may be administered in a dose ranging from about 50 mg/kg to about 500 mg/kg. L-ornithine phenylacetate may be administered in a dose ranging from about 100 mg/kg to about 250 mg/kg. In one example, L-ornithine phenylacetate is administered in a dose of about 50 mg/kg. In another example, L-ornithine phenylacetate is administered in a dose of about 200 mg/kg.

In some embodiments, the L-ornithine phenylacetate for use in treating hyperammonia is administered in an amount from about 0.1 g to about 50.0 g per day, from about 1.0 g to about 40.0 g per day, from about 5.0 g to about 30.0 g per day, from about 10.0 g to about 25.0 g per day, or from about 15.0 g to about 25.0 g per day. In some embodiments, the pharmaceutical formulation is for administration at least once a day. In some further embodiments, the pharmaceutical formulation is for administration 2 or 3 times a day. In one embodiment, the formulation is for thrice daily oral administration.

L-ornithine phenylacetate is administered as a single dose in an amount from about 2.0 g to about 9.0 g. In some embodiments, L-ornithine phenylacetate is administered as a single dose in an amount from about 5.0 g to about 8.0 g. In some such embodiments, the pharmaceutical formulation containing such amount of L-ornithine phenylacetate is in two or more unit dosage forms. For example, some embodiments comprise administering 3 to 6 unit dosage forms each comprising from about 0.75 g to about 2.0 g of L-ornithine phenylacetate, or about 3 to 5 unit dosage forms each comprising from about 1.0 g to about 1.5 g of L-ornithine phenylacetate. Some embodiments comprise administering 4 unit dosage forms each comprising about 1.25 g of L-ornithine phenylacetate. Some embodiments comprise administering 5 unit dosage forms each comprising about 1.0 g of L-ornithine phenylacetate. In one embodiment, the pharmaceutical formulation is administered three times a day. For example, where multiple unit dosage forms are administered at a time, the multiple unit dosage administration is repeated three times a day.

In some embodiments, the plasma Cmax of L-ornithine is from about 10 mg/L to about 60 mg/L, from about 12 mg/L to about 50 mg/L, or from about 15 mg/L to about 40 mg/L. In some further embodiments, the plasma Cmax of L-ornithine is from about 18.0 mg/L to about 35.0 mg/L.

In some embodiments, the plasma Cmax of metabolite phenylacetic acid ranges from about 15 mg/L to about 120 mg/L. In some such embodiments, the plasma Cmax of metabolite phenylacetic acid is from about 15 mg/L to about 55 mg/L. In some such embodiments, the plasma Cmax of metabolite phenylacetic acid is from about 20 mg/L to about 100 mg/L. In some such embodiments, the plasma Cmax of metabolite phenylacetic acid is from about 40 mg/L to about 90 mg/L. In some further embodiments, the plasma Cmax of metabolite phenylacetic acid is from about 50 mg/L to about 90 mg/L.

In some embodiments, the plasma Cmax of metabolite phenylacetylglutamine ranges from about 15 mg/L to about 75 mg/L. In some such embodiments, the plasma Cmax of metabolite phenylacetylglutamine is from about 15 mg/L to about 35 mg/L. In some such embodiments, the plasma Cmax of metabolite phenylacetylglutamine is from about 20 mg/L to about 60 mg/L. In some such embodiments, the plasma Cmax of metabolite phenylacetylglutamine is from about 25 mg/L to about 50 mg/L. In some further embodiments, the plasma Cmax of metabolite phenylacetylglutamine is from about 30 mg/L to about 45 mg/L.

In some embodiments, the pharmaceutical formulation provides conversion of phenylacetate to phenylacetylglutamine over 24 hours of greater than about 30%, greater than about 40%, greater than about 50%, greater than about 60%, greater than about 70%, greater than about 80%, or greater than about 90%. In some further embodiments, the pharmaceutical formulation provides conversion of phenylacetate to phenylacetylglutamine over 24 hours of greater than about 80%.

### Definitions

Unless defined otherwise, all technical and scientific terms used herein have the same meaning as is commonly understood by one of ordinary skill in the art. The use of the term "including" as well as other forms, such as "include", "includes," and "included," is not limiting. The use of the term "having" as well as other forms, such as "have", "has," and "had," is not limiting. As used in this specification, whether in a transitional phrase or in the body of the claim, the terms "comprise(s)" and "comprising" are to be interpreted as having an openended meaning. That is, the above terms are to be interpreted synonymously with the phrases "having at least" or "including at least." For example, when used in the context of a process, the term "comprising" means that the process includes at least the recited steps, but may include additional steps. When used in the context of a compound, composition, formulation, or device, the term "comprising" means that the compound, composition, formulation, or device includes at least the recited features or components, but may also include additional features or components.

As used herein, common organic abbreviations are defined as follows:
- AUC: Area under the curve
- AUC_{0→t}: Area under the concentration vs. time curve from time = 0 (zero) to time of last quantifiable concentration
- AUC_{0→∞}: Area under the plasma concentration with time curve extrapolated to infinite time
- C₁₂: Drug concentration at 12 hr after drug administration
- CL: Coat Level
- Cmax: Maximum plasma concentration
- HPMC: Hydroxypropylmethylcellulose
- hr: Hour(s)
- IR: Immediate release
- MCC: Microcrystalline cellulose
- ORN: Ornithine
- PAA: Phenylacetic acid (or the conjugate base phenylacetate)
- PAGN: Phenylacetylglutamine
- PD: Pharmacodynamic
- PK: Pharmacokinetic
- SEM: Scanning Electron Micrographs

The term "immediate release" as used herein, has its ordinary meaning as understood by those skilled in the art and thus includes, by way of non-limiting example, release of a drug from a dosage form in a relatively brief period of time after administration.

The term "controlled release" and the term "extended release" as used herein, each has its ordinary meaning as understood by those skilled in the art and thus includes, by way of non-limiting example, controlled release of a drug from a dosage form over an extended period of time. For example, controlled release or extended release formulations are those that have a release rate that is substantially longer than that of a comparable immediate release form. The two terms can be used interchangeably.

The term "phenylacetic acid" as used herein, is also known as benzeneacetic acid or 2-phenylacetic acid). It has the following chemical structure:

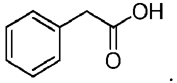

The term "phenylacetate" as used herein, refers to the anionic form of phenylacetic acid with the following chemical structure:

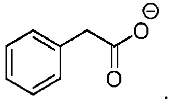

The term "L-ornithine phenylacetate" as used herein, refer to a compound consisting of L-ornithine cation and phenylacetate anion. It has the following chemical structure:

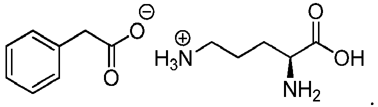

The term "phenylacetylglutamine" as used herein, refers to the product formed by the conjugation of phenylacetic acid and glutamine. It is a common metabolite that can be found in human urine. It has the following chemical structure:

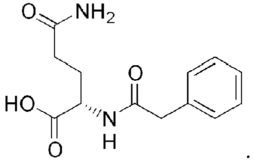

The term "percentage of coat level" as used herein, refers to the weight percentage of a particle coating in the total mass of the finished particle. For example, 15% CL extrudate means the coating of the particle constitutes about 15% by weight of the total mass of the finished particle.

As used herein, the term "percent conversion of phenylacetate to phenylacetylglutamine over 24 hours" refers to the mass percent of phenylacetate administered to a patient that is converted to phenylacetylglutamine collected over 24 hours in the urine.

The term "pharmaceutically acceptable carrier" or "pharmaceutically acceptable excipient" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions or formulations is contemplated. Supplementary active ingredients can also be incorporated into the compositions or formulations. In addition, various adjuvants such as are commonly used in the art may be included. These and other such compounds are described in the literature, e.g., in the Merck Index, Merck & Company, Rahway, NJ. Considerations for the inclusion of various components in pharmaceutical compositions are described, e.g., in Gilman et al. (Eds.) (1990); Goodman and Gilman's: The Pharmacological Basis of Therapeutics, 8th Ed., Pergamon Press.

The term "pharmaceutically acceptable salt" refers to salts that retain the biological effectiveness and properties of the compounds of the preferred embodiments and, which are not biologically or otherwise undesirable. In many cases, the compounds of the preferred embodiments are capable of forming acid and/or base salts by virtue of the presence of amino and/or carboxyl groups or groups similar thereto. Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese, aluminum, and the like; particularly preferred are the ammonium, potassium, sodium, calcium and magnesium salts. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. Many such salts are known in the art, as described in WO 87/05297, Johnston et al., published September 11, 1987.

"Subject" as used herein, means a human or a non-human mammal, e.g., a dog, a cat, a mouse, a rat, a cow, a sheep, a pig, a goat, a non-human primate or a bird, e.g., a chicken, as well as any other vertebrate or invertebrate.

"Treat," "treatment," or "treating," as used herein refers to administering a pharmaceutical composition/formulation for prophylactic and/or therapeutic purposes. The term "prophylactic treatment" refers to treating a patient who is not yet suffering from a disease, but who is susceptible to, or otherwise at risk of, a particular liver disease, whereby the treatment reduces the likelihood that the patient will develop a liver disease. The term "therapeutic treatment" refers to administering treatment to a patient already suffering from a liver disease.

The compositions or formulations described herein are provided in unit dosage form. As used herein, a "unit dosage form" is a composition/formulation containing an amount of a compound that is suitable for administration to an animal, preferably mammal subject, in a single administration, according to good medical practice. The preparation of a single or unit dosage form however, does not imply that the dosage form is administered once per day or once per course of therapy, or that the unit dosage form contains all of the dose to be administered at a single time. Such dosage forms are contemplated to be administered once, twice, thrice or more per day, and may be given more than once during a course of therapy, though a single administration is not specifically excluded. In addition, multiple unit dosage forms may be administered at substantially the same time to achieve the full dose intended (e.g., two or more tablets may be swallowed by the patient to achieve a complete dose). The skilled artisan will recognize that the formulation does not specifically contemplate the entire course of therapy and such decisions are left for those skilled in the art of treatment rather than formulation.

### Methods of Manufacturing

The key challenges in the development of the oral formulation of L-ornithine phenylacetate include the high dose of the API (for example, about 20 g/day); the high API solubility; the short API half-life (for example, phenylacetic acid has a short half-life of about 90 minutes); the taste/odor masking, the palatability and alcohol resistance. In particular, the high solubility of L-ornithine phenylacetate renders the controlled release formulation very challenging due to the fast dissolution of the API. Extreme high osmotic pressures build up inside the coating and can rupture the coating membrane if it is not strong enough to withstand the forces.

As discussed herein, the present application discloses a multi-particulate controlled release oral formulation system (not according to the invention) to address some or all of the key challenges. The system comprises a core and one or more coating layers on the core.

### Core Composition and Processes

The core comprises the API - L-ornithine phenylacetate and one or more excipients. In some instances, L-ornithine phenylacetate is mixed with the core excipient(s) to form granules. In some other instances, L-ornithine phenylacetate is applied to an inert core (consists of excipients only) as a layer, for example, by spray layering. In some other instances, L-ornithine phenylacetate is combined with ion exchange resins in particle form.

The granulation process includes the step of combining L-ornithine phenylacetate with excipients in fluid bed or high-shear granulator. This process yields rough, porous, irregular-shaped particles. The use of Glatt specialty granulation technology offers an improvement to the standard granulation process, resulting in the formation of uniform spherical granules with a high drug load.

Alternatively, an extrusion spheronization process can be used, including the steps of combining L-ornithine phenylacetate and excipients to make plastic dough, extruding, spheronizing the extrudate. This process results in the yield of somewhat spherical particles.

The spray layering process includes the step of building up a layer of L-ornithine phenylacetate on an inert spherical core via fluid bed coating operation. This process yields spherical particles. L-ornithine phenylacetate may first be dissolved in a solvent optionally comprising a hydrophilic polymer to form L-ornithine phenylacetate coating solution. Then, such coating solution is sprayed on an inert core. Non-limiting examples of hydrophilic polymers includes compounds selected from acrylic or methacrylic acid polymers or copolymers, alkylvinyl polymers, hydroxyalkyl celluloses, carboxyalkyl celluloses, polysaccharides, dextrins, pectins, starches and derivatives, natural or synthetic gums. The hydrophilic polymer may be hydroxypropylmethylcellulose (HPMC). Non-limiting examples of the inert core described herein includes microcrystalline cellulose spheres such as Celphere^{™} from Asahi-Kasei and sugar/starch spheres such as SUGLETS^{®} from Colorcon.

The melt-spray-congeal process includes the step of embedding L-ornithine phenylacetate in a molten waxy matrix; spraying and cooling to yield the spherical particles.

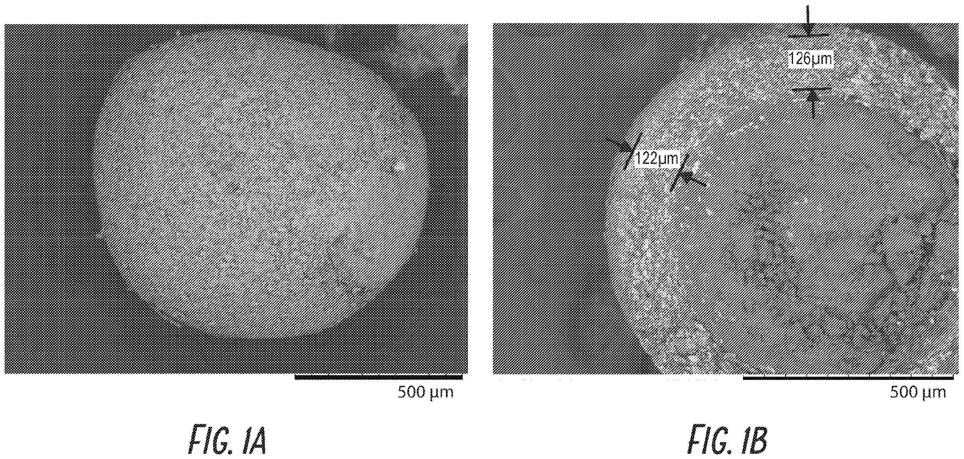

The granules produced by the standard granulation process may not be an adequate substrate for coating. Particles produced by granulation are inherently porous, irregularly shaped particles with a rough surface. In these cases, processes such as spray layering and extrusion spheronization may be used to provide more uniform core granules. *See* **FIGs. 1A** and **1B.** FIG. 1B shows that the final particle with a coating thickness between 122 µm and 126 µm.

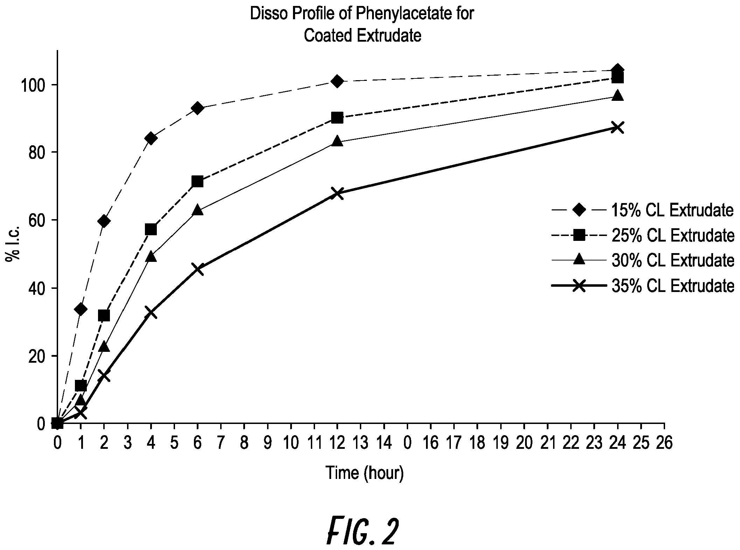

**FIG. 2** illustrates the dissolution profile of phenylacetate of the various coated ethyl cellulose extrudates. Specifically, "% l.c." refers to percent label claim. It is the percentage of the total dose that is dissolved at a given time in the dissolution vessel. The dissolution test was carried out as described in the USP, General Chapter 711, using Apparatus 2 Paddle Method. **FIG. 2** shows the dissolution profiles of four different coated extrudates with different percentage of coat level (CL) - 15% CL, 25% CL, 30% CL and 35% CL extrudate. The dissolution profiles suggest that the higher the percentage of coating used, the slower the release rate is. These *in vitro* data suggest that the BID dosing profile is feasible.

### Coating Composition and Processes

As discussed herein, the multi-particulate controlled release oral formulation system comprises one or more coating layers on the core. The coating composition may comprise a cellulose derivative, for example, ethyl cellulose to provide controlled-release. Alternatively, polymethacrylates (Eudragits) can also be used either in dispersions or solvent based. Other polymers may be used in the present application include, but not limited to alkyl cellulose, hydroxyalkyl cellulose, carboxyalkyl cellulose, cellulose acetate, alkylvinyl polymers, acrylic acid polymers or copolymers, polyvinyl pyrrolidine, polyvinyl acetate, polymethacrylate, natural or synthetic gums.

Aqueous-based coating formulation may be problematic. Because of the high water solubility of L-ornithine causes the API to dissolve in the coating during the coating process, effectively increasing the coating porosity. A solvent-based coating is a good alternative approach.

The coating layer can be applied to the core via various processes. The fluid bed coating process includes the step of recirculating particles in streams of air; spraying coating agent onto particles to gradually build up the coating. The spray step can be performed via top-spray, bottom-spray, tangential spray, etc. Alternatively, the coating layer can be applied on the core via Wurster coating process. The Wurster coating process is a variant of bottom-spray fluid bed coating and is widely used in pharmaceutical applications.

Two or more coating layers may be used. The multi coating layers may include a seal coating, a control coating, and/or an enteric coating. The additional coating may add or augment the product properties, for example, improving alcohol resistance. For example, the enteric coating may comprise, for example, acrylic and methacrylic acids polymers (Eudragit^{®}) or copolymer or cellulose derivatives, such as cellulose acetophthalate.

Other factors that may affect final product properties include, but are not limited to coating thickness, plasticizer type and concentration, pore former or other additives and process variables (spray rate, drying time, temperatures, etc.). The average coating thickness may be from about 10 µm to about 500 µm, from about 25 µm to about 250 µm, or from about 50 µm to about 125 µm.

The controlled release oral formulation may be prepared by combining L-ornithine phenylacetate particles with different coatings. This facilitates the adjustment of the pharmacokinetics profile of the final controlled release formulation to achieve the desired sustainable exposure.

Some examples of substances that can serve as pharmaceutically-acceptable carriers or excipients thereof, are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and oil of theobroma; polyols such as propylene glycol, glycerine, sorbitol, mannitol, and polyethylene glycol; alginic acid; emulsifiers, such as the TWEENS; wetting agents, such sodium lauryl sulfate; coloring agents; flavoring agents; tableting agents, stabilizers; antioxidants; preservatives; pyrogen-free water; isotonic saline; and phosphate buffer solutions.

Non-limiting examples of oral formulation tablet excipients or carriers comprise conventional pharmaceutically-compatible adjuvants as inert diluents, such as calcium carbonate, sodium carbonate, mannitol, lactose and cellulose; binders such as starch, gelatin and sucrose; disintegrants such as starch, alginic acid and croscarmelose; lubricants such as magnesium stearate, stearic acid and talc. Glidants such as silicon dioxide can be used to improve flow characteristics of the powder mixture. Coloring agents, such as the FD&C dyes, can be added for appearance. Sweeteners and flavoring agents, such as aspartame, saccharin, menthol, peppermint, and fruit flavors, are useful adjuvants for chewable tablets. Capsules typically comprise one or more solid diluents disclosed above.

In some embodiments, the oral formulation can also be in the form of pellets or free-flow powders. In particular, the pellets or free-flow powder can be further packed into sachets for oral administration. In some embodiments, the pellets or free-flow powder can be administered in conjunction with food and/or beverage in order to improve patient compliance and tolerability. For example, the pellets or powder in the sachet can be mixed with a beverage such as juice or water to form a suspension. Alternatively, the pellets or powder in the sachet can be mixed with food. Preferably, the size of particles is not too granular such that it is undesirable to be administered orally. The palatable oral sachet formulation may also comprise conventional pharmaceutical compatible adjuvants, excipients or carriers, including those commonly used in the oral tablet formulation as discussed herein.

In some embodiments, the oral formulation of the invention provides for lower doses than previously expected. For example, RAVICTI^{®} (glycerol phenylbutyrate, a pre-prodrug of phenylacetate) was found in clinical studies at a dose of 6 mL (delivering about 1.02 g/mL of phenylbutyrate) twice daily to lower the incidence of hepatic encephalopathy events. Both the immediate release and the controlled release oral pharmaceutical formulations of L-ornithine phenylacetate described herein provide similar percentage of PAGN urinary excretion, permitting use of substantially lower API doses, compared to RAVICTI^{®} or other phenylacetate formulations.

### EXAMPLES

The following examples, including experiments and results achieved, are provided for illustrative purposes only.

### Example 1 - Preparation of Coated Controlled Release Formulations (example not according to the invention)

Three controlled release oral pharmaceutical formulations of L-ornithine phenylacetate (Formulations 3, 5 and 6) were prepared. Formulations 3 and 6 comprise sugar spheres ("Suglets") onto which a mixture of L-ornithine phenylacetate and hydroxypropylmethylcellulose (HPMC) in aqueous solution has been spray-layered up to an approximate weight percentage of 35%. After drying, these spray-layered beads or pellets were further coated using a solution of ethyl cellulose ('ETHOCEL^{™}") dissolved in organic solvents. By adjusting the amount of ethyl cellulose coating applied, the release properties of the pellets may be adjusted to yield the desired release profile. This profile may be measured *in vitro,* such as by the dissolution results, and also may be assessed by *in vivo* techniques in which the pharmacokinetic profile of the drug in plasma (or other biological locations, such as urine) is assessed. Formulation 3 is described as a 25% coat level bead, and Formulation 6 as a 15% coat level bead. The excipient talc may also be added to the ethyl cellulose.

The preparation of Formulation 5 began in a similar fashion to Formulations 3 and 6, except that instead of an ethyl cellulose coating, a coating comprised of Eudragit NM 30 D was applied using an aqueous dispersion of the NM 30 D polymer. Eudragit NM 30 D is a neutral copolymer based on ethyl acrylate and methyl methacrylate. The amount of coating was approximately 70% weight gain over the original weight of the drug-layered bead. In addition to the Eudragit polymer, talc was used in the coating dispersion to yield a product with suitable properties.

### Example 2 - Pharmacokinetic Studies in Dogs

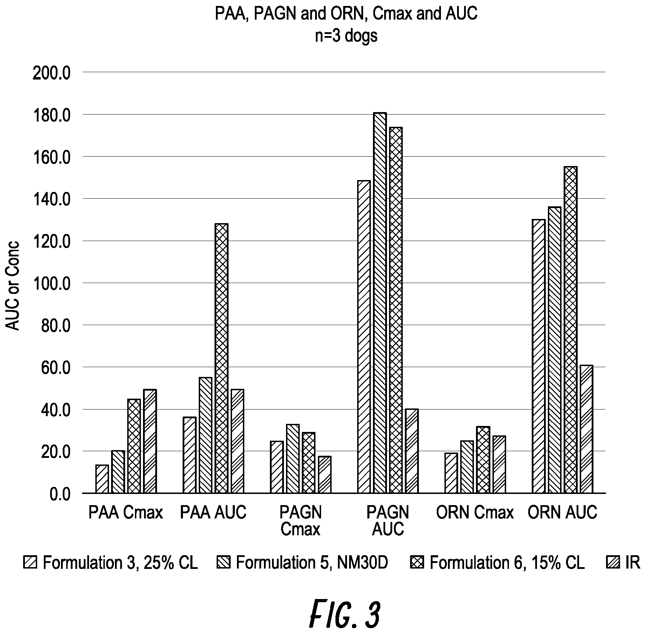

A pharmacokinetic study of the controlled-release oral formulations of L-ornithine phenylacetate was conducted in dogs. 6 dogs in two sets of three were used in the study. The controlled-release formulations (Formulations 3, 5 and 6 as described in Example 1) were administered orally at a dose of 200 mg/kg. An immediate release formulation of L-ornithine phenylacetate in water was administered orally at the dose of 50 mg/kg. The AUC and plasma Cmax of analyte L-ornithine and phenylacetate metabolites phenylacetylglutamine (PAGN) and phenylacetic acid (PAA) are illustrated in **FIG. 3****.** All the controlled-release formulations produced comparable PAGN and ORN pharmacokinetics and the ratio of plasma Cmax to AUC is equal for the controlled release formulations with respect to PAA and PAGN.

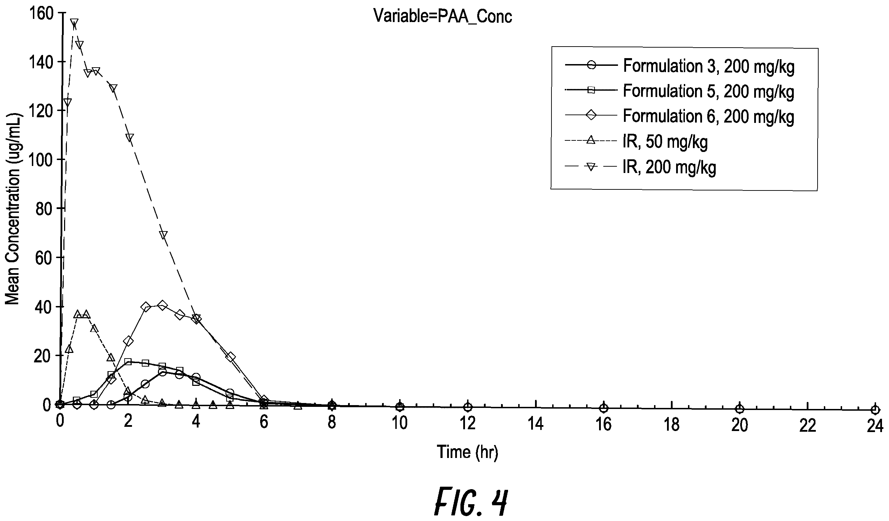

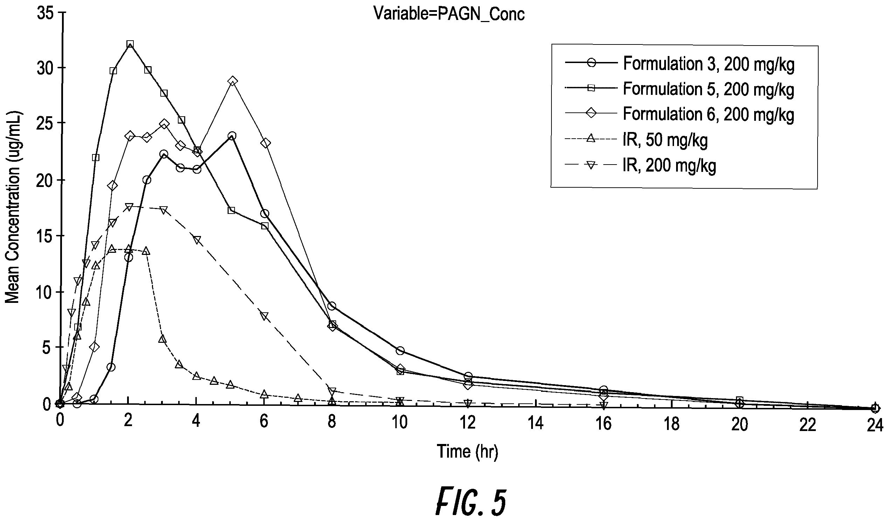

*In vivo* PK data of metabolite PAA and *in vivo* surrogate PD data of metabolite PAGN in animal models were collected and demonstrated in **FIGs. 4** and **5** where the three controlled-release formulations (Formulations 3, 5 and 6) were administered at a dose of 200 mg/kg and two immediate-release formulations were administered at the dose of 50 mg/kg and 200 mg/kg respectively.

### Example 3 - Preparation of Large Pellets of Coated Controlled Release Formulations (example not according to the invention)

Three controlled release oral pharmaceutical formulations of L-ornithine phenylacetate (Formulations A, B and C) were prepared following similar procedure described in Example 1. Sugar spheres with an average diameter between 500 and 600 µm were used as core. Then, a mixture of L-ornithine phenylacetate and hydroxypropylmethylcellulose (HPMC) in aqueous solution has been spray-layered onto the sugar spheres. In each case, the excipient talc was also used in forming the layered core pellets.

After drying, these layered core pellets were further coated with an extended release polymer selected from ethyl cellulose or Eudragit NM 30 D. The components of Formulations A, B, and C are summarized in Table 1 below. The final pellets in Formulations A, B, and C contain 15%, 30% or about 41% extended release coating respectively, relative to the total weight of the pellets. The average diameter of the final large pellets is between about 650 µm and 950 µm, for example, about 800 µm.

| **Table 1.** | | | |
|---|---|---|---|
| Component | Quantity (g/dose) | | |
| | Formulation A | Formulation B | Formulation C |
| **Drug Layered Core Pellets (g)** | | | |
| L-ornithine phenylacetate | 10.00 | 10.00 | 10.00 |
| Sugar Spheres (500-600 µm) | 17.57 | 17.57 | 17.57 |
| Talc | 0.50 | 0.50 | 0.50 |
| Hydroxypropylmethylcellulose | 0.50 | 0.50 | 0.50 |
| Total (Dry weight) Core Pellets | 28.57 | 28.57 | 28.57 |

| **Extended Release Coating (g)** | | | |
|---|---|---|---|
| Ethyl cellulose | 4.54 | 11.02 | NA |
| Dibutyl Sebacate | 0.50 | 1.22 | NA |
| Eudragit NM 30D | NA | NA | 11.42 |
| Talc | NA | NA | 8.57 |
| Total Drv Weight per Unit Dose | 33.6 | 40.8 | 48.6 |

### Example 4 - Preparation of Small Pellets of Coated Controlled Release Formulations (example not according to the invention)

A large scale preparation of two controlled release oral pharmaceutical formulations of L-ornithine phenylacetate (Formulations D and E) were carried out, following similar procedures described in Example 1. First, a HPMC solution was prepared by dissolving 0.765 kg HPMC in 27.4 kg water. Then, 15.2 kg L-ornithine phenylacetate was dissolved in this HPMC aqueous solution. 0.765 kg Talc was then suspended into this mixture. The resulting L-ornithine phenylacetate coating suspension was sprayed onto 5 kg microcrystalline cellulose spheres with the average diameter between 100 µm and 200 µm using a fluid bed coating process to form the core pellets. The final core pellets comprises about 70% w/w of L-ornithine phenylacetate. Interestingly, during the spraying process, it was observed that a crystalline form of L-ornithine phenylacetate was precipitating out of the L-ornithine phenylacetate coating suspension. The crystalline structure of the precipitate was determined by X-Ray Powder Diffraction (XRPD) analysis and the result confirmed it was Form II, previously reported in PCT Pub. No. WO2010/115055 A1.

To prepare Formulation D, ethyl cellulose coating was applied to a polymer weight gain of 80% w/w. For example, if the core pellets for a batch weighed 1000 g, 800 g of ethyl cellulose would be sprayed onto the core pellets. To prepare Formulation E, Eudragit NM 30 D coating was applied to a polymer weight gain of 185% w/w. For example, if the core pellets for a batch weighed 1000 g, 1850 g of Eudragit NM 30 D would be sprayed onto the core pellets. Two batches of Formulation D smaller pellets were prepared.

The components of Formulations D and E are summarized in Table 2 below. The average diameter of the final small pellets is between about 300 µm and about 600 µm, for example, between about 300 µm and about 425 µm for pellets of Formulation D and between 425 µm and about 500 µm for pellets of Formulation E.

| **Table 2.** | | |
|---|---|---|
| Component | Formulation D | Formulation E |
| **Core Pellet (g)** | | |
| L-Ornithine Phenylacetate | 10 | 10 |
| Microcrystalline Cellulose Spheres (Celphere CP102, 100-200 µm) | 3.3 | 3.3 |
| Talc | 0.5 | 0.5 |
| Hydroxypropyl Methylcellulose (E5) | 0.5 | 0.5 |
| Total (Dry weight) Core Pellets | 14.3 g | 14.3 g |

| **Extended Release Pellets (g)** | | |
|---|---|---|
| Ethyl cellulose (80% polymer weight gain) | 11.4 | NA |

| | | |
|---|---|---|
| Dibutyl Sebacate | 1.3 | NA |
| Eudragit NM 30 D (185% polymer weight gain) | NA | 26.5 |
| Talc | NA | 19.9 |
| Total (Dry Weight) of Coated Pellets | 27 g | 60.7 g |

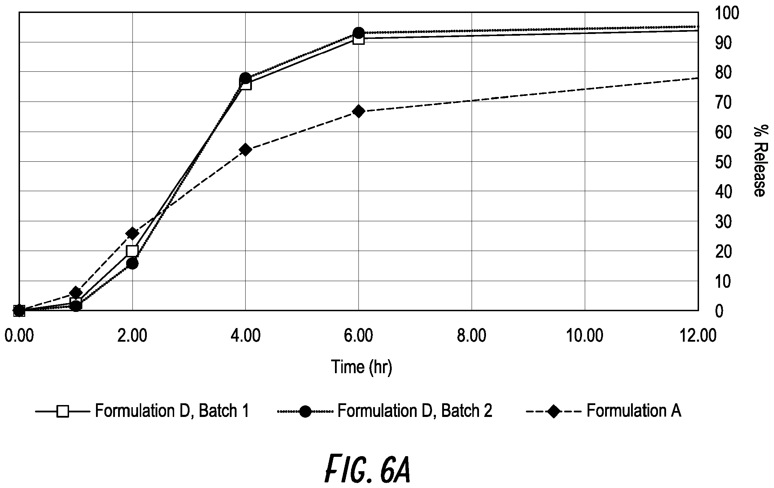

**FIG. 6A** illustrates the dissolution profiles of two batches of small pellets of Formulation D and the large pellets of Formulation A, all of which utilized ethyl cellulose for providing the extended release. The dissolution test was carried out as described in the USP, General Chapter 711, using Apparatus 2 Paddle Method, 75 rpm at 37°C in water. It was observed that the release profile of L-ornithine phenylacetate in Formulation D small pellets was about 18% to 20% in 2 hours, about 76% to 78% in 4 hours, and about 91% to 93% in 6 hours. In comparison, the release profile of L-ornithine phenylacetate in Formulation A large pellets was about 27% in 2 hours, about 54% in 4 hours, and about 68% in 6 hours.

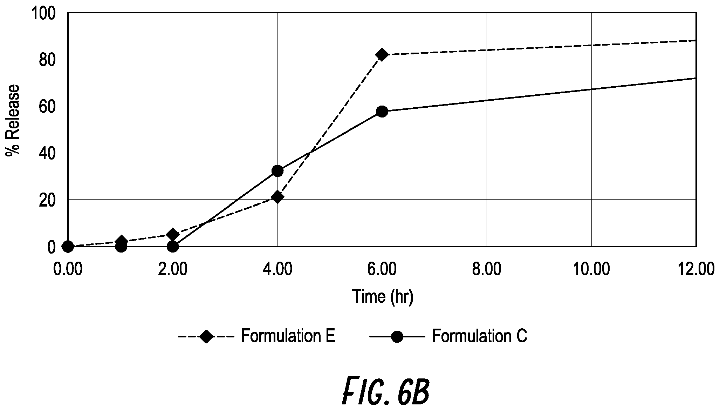

**FIG. 6B** illustrates the dissolution profiles of small pellets of Formulation E and the large pellets of Formulation C, both of which utilized Eudragit NM 30 D for providing the extended release. The dissolution test was carried out as described in the USP, General Chapter 711, using Apparatus 2 Paddle Method, 75 rpm at 37°C in water. It was observed that the release profile of L-ornithine phenylacetate in Formulation E small pellets was about 8% in 2 hours, about 20% in 4 hours, and about 82% in 6 hours. In comparison, the release profile of L-ornithine phenylacetate in Formulation C large pellets was less than about 5% in 2 hours, about 32% in 4 hours, and about 58% in 6 hours.

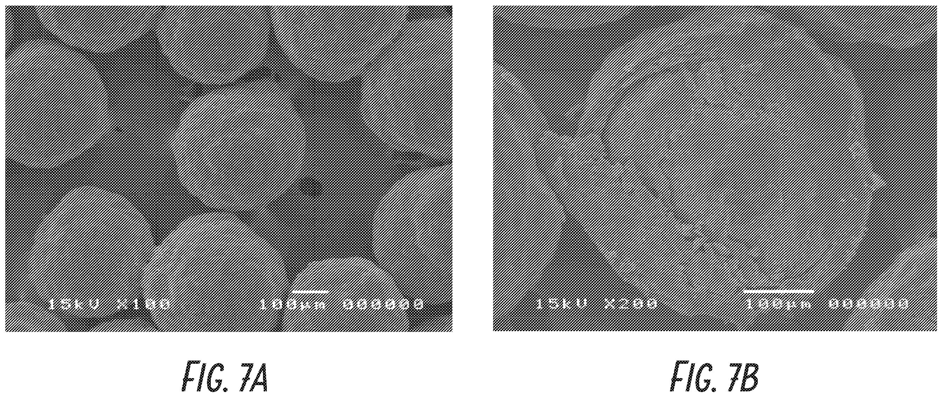

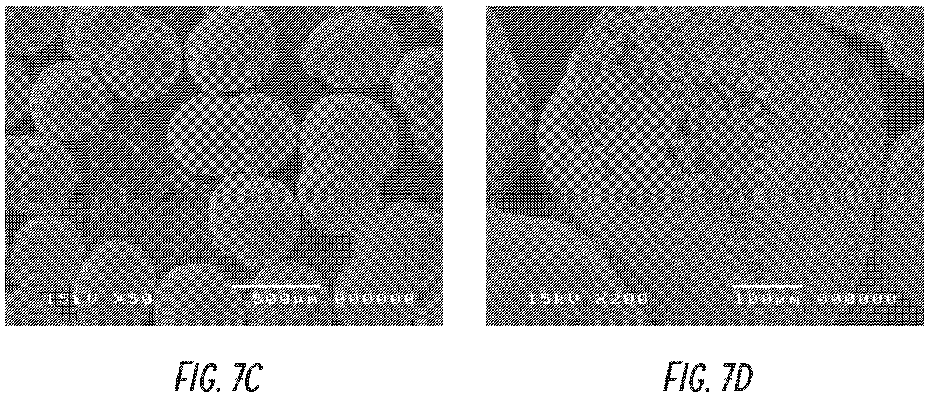

The Scanning Electron Micrographs (SEM) images of the small pellets of Formulation D are illustrated in **FIGs. 7A** and **7B** in different magnitudes. The Scanning Electron Micrographs (SEM) images of the small pellets of Formulation E are illustrated in **FIGs. 7C** and **7D** in different magnitudes.

### Example 5 - Pharmacokinetic Studies in Humans

An open-label, five-treatment, five-period single-dose crossover Phase 1 human clinical study was conducted to evaluate the pharmacokinetics of phenylacetic acid and phenylacetylglutamine after a single dose of three extended-release oral dosage forms of L-ornithine phenylacetate in comparison to RAVICTI^{®} (glycerol phenylbutyrate), a prodrug of phenylacetic acid. The study also compared the pharmacokinetics and safety of single doses of the three extended-release oral dosage forms of L-ornithine phenylacetate in comparison to a single dose of an immediate release oral solution of L-ornithine phenylacetate.

The five treatments are listed as follows: each of Treatment A, B, and C refers to a single oral dose of 10 g Formulations A, B and C (each is an equivalent of about 5 g PAA); Treatment D refers to a single oral dose of 6 mL RAVICTI^{®} (equivalent of about 5 g PAA); Treatment E refers to a single oral dose of an immediate release formulation of 5 g L-ornithine phenylacetate (equivalent of about 2.5 g PAA).

The primary objective is to assess the plasma profiles and pharmacokinetics of phenylacetic acid (a potent ammonia scavenger), ornithine and phenylacetylglutamine (the end-product responsible for clearing ammonia) following a single oral dose of three extended-release formulations of L-ornithine phenylacetate in comparison to an oral solution of L-ornithine phenylacetate and a prodrug of phenylacetic acid (glycerol phenylbutyrate, RAVICTI^{®}) in healthy human subjects. The secondary objective is to determine the safety, tolerability, and palatability of three extended-release formulations in healthy subjects.

Eligible male or female adult healthy subjects were enrolled to first receive four treatments (Treatments A-D) over 4 dosing periods in a crossover fashion using a balanced 4x4 Latin Square design with an at least 7-day washout interval between treatments followed by receiving Treatment E for all subjects in the fifth (last) dose period after a minimum 7-day washout interval. Following dosing in each dose period, subjects underwent serial blood and urine sampling up to 24 hrs post dose for PK assessment.

### PK Assessments

In the dose periods where Treatment A, B, C, or D was administered (the ER formulations of OCR-002 or RAVICTI^{®}), venous blood samples (5 mL each) were collected at the following time points: immediately (within 15 mins) prior to dosing, and then at 0.5, 1, 1.5, 2, 2.5, 3, 3.5, 4, 5, 6, 8, 10, 12, 16, 20, and 24 hrs post dose. In the dose period where the immediate release formulation of L-ornithine phenylacetate was administered (Period 5), venous blood samples (5 mL each) were collected at the following time points: immediately (within 15 mins) prior to dosing, and then at 0.25, 0.5, 0.75, 1, 1.5, 2, 2.5, 3, 3.5, 4, 4.5, 5, 6, 7, 8, 10, and 12 hrs post-dose.

In addition, urine samples were collected at the following time intervals: within 1 hour prior to dosing (spot sample), and then cumulatively over the intervals of 0-4, 4-8, 8-12, and 12-24 hrs post dose. Plasma samples were separated by centrifugation within 1 hour of blood collection and stored at approximately -80 °C until analyzed. The total urine volume for each collection interval were measured and recorded and aliquots of urine were stored at approximately -80 °C until analyzed.

### Bioanalytical Methods

Plasma samples were analyzed for concentrations of phenylacetic acid (PAA), phenylacetylglutamine (PAGN) and ornithine (ORN) using a validated LC-MS/MS method. All urine samples were analyzed for concentrations of PAGN using a validated LC-MS/MS method.

### Endpoints

Pharmacokinetics: Plasma concentration vs. time profiles of phenylacetate, ornithine, and phenylacetylglutamine following a single oral dose of each of the study drug were analyzed by noncompartmental PK methods. Pharmacokinetic parameters that were determined include Cₘₐₓ, tₘₐₓ, AUC₀₋ₜ, AUC_{0-∞}, C₁₂ and t_{1/2}. The amount of PAGN excreted in urine and the percent of PAA dose excreted in urine as PAGN over each collection interval and the entire 24 hr interval were also determined.

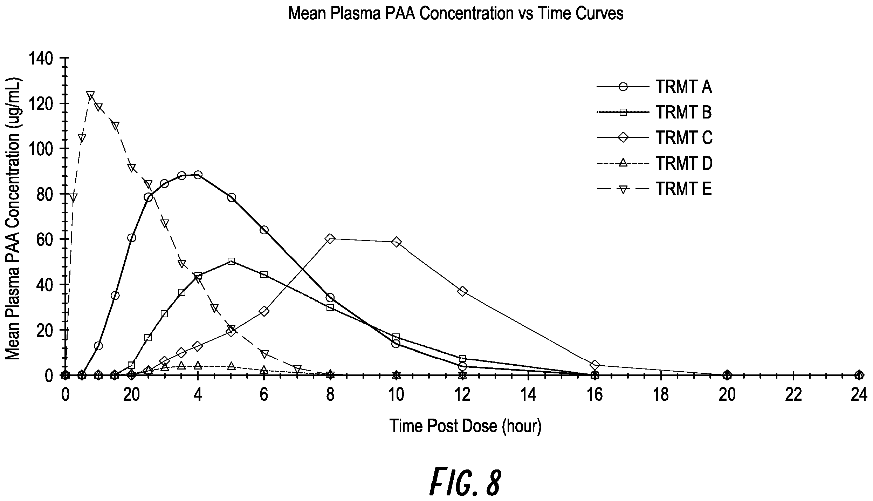

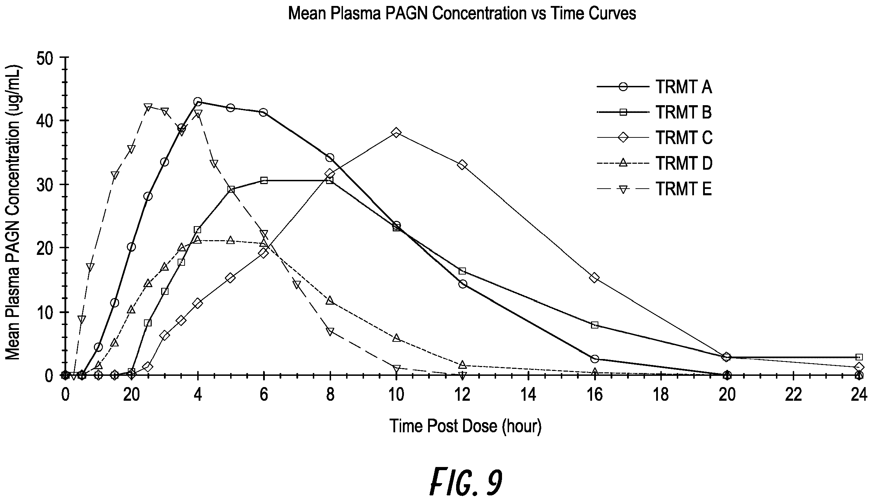

**FIG. 8** and **FIG. 9** illustrate the mean plasma profiles of PAA and PAGN, respectively, from this Phase 1 study. **FIG. 8** demonstrates the Mean Plasma PAA concentration vs. time curves following administration of a single oral dose of the controlled-release formulations in comparison to the immediate-release solution and glycerol phenylbutyrate. **FIG. 9** demonstrates the Mean Plasma PAGN concentration vs. time curves following administration of a single oral dose of the controlled-release formulations in comparison to the immediate-release solution and glycerol phenylbutyrate.

The mean maximum concentration (Cmax) of plasma PAA from three extended-release formulations ranged from approximately 50 to 90 µg/mL occurring at various time points over 4 to 9 hours after dosing. For comparison, RAVICTI^{®} produced a mean plasma PAA Cmax of approximately 10 µg/mL at 4 to 6 hours after dosing. The plasma PAA data after a single oral dose of 6mL RAVICTI^{®} are consistent with published data in healthy subjects. In addition, PAA exposure with the extended-release formulations of L-ornithine phenylacetate showed lower inter-subject variability than RAVICTI^{®}.

Plasma profiles of PAGN, the end-product of ammonia scavenging, also demonstrated a similar pattern as the PAA profiles. The mean Cmax of plasma PAGN from the three extended-release formulations of L-ornithine phenylacetate ranged from approximately 30 to 45 µg/mL occurring at various time points over 4 to 10 hours after dosing. For comparison, RAVICTI^{®} produced a mean plasma PAGN Cmax of about 20 to 25µg/mL at approximately 5 hours. These data are also consistent with the published data in healthy subjects.

The total urinary excretion data of PAGN over 24 hours is summarized in Table 3 below. The mean PAGN excretion amount was comparable for Treatment A through C, each with about 80% PAA converted to PAGN excretion over 24 hours. In contrast, Treatment D with RAVICTI^{®} only showed about 40% conversion efficiency compared to Treatment A through C at approximately the same molar PAA dose (in the case of RAVICTI^{®}, PAA is provided from the glycerol phenylbutyrate prodrug). It was surprisingly observed that the immediate release formulation Treatment E also exhibited about 80% conversion efficiency, which provided a similar mean PAGN excretion amount at approximately half the molar dose of PAA administered in the RAVICTI^{®} arm.

**Table 3.**

| **Treatment** | **Statistics** | **Total PAGN Amount Excreted over 24 hours (G)** | **PAA Equivalents Excreted over 24 hours (G)** | **Percent PAA Dose Excreted over 24 hours (%)** |
|---|---|---|---|---|
| **A** | N | 12 | 12 | 12 |
| | Mean | 8.26 | 4.26 | 83.8 |
| | Median | 8.47 | 4.37 | 86.0 |
| | %CV | 14.7 | 14.7 | 14.7 |
| **B** | N | 12 | 12 | 12 |
| | Mean | 7.77 | 4.00 | 78.9 |
| | Median | 7.77 | 4.00 | 78.8 |
| | %CV | 10.7 | 10.7 | 10.8 |
| **C** | N | 12 | 12 | 12 |
| | Mean | 8.53 | 4.39 | 86.6 |
| | Median | 8.39 | 4.33 | 85.3 |
| | %CV | 11.3 | 11.3 | 11.3 |
| **D** | N | 12 | 12 | 12 |
| | Mean | 4.11 | 2.12 | 42.7 |
| | Median | 4.06 | 2.09 | 42.1 |
| | %CV | 24.9 | 24.9 | 24.9 |
| **E** | N | 12 | 12 | 12 |
| | Mean | 4.01 | 2.07 | 81.5 |
| | Median | 4.14 | 2.13 | 84.0 |
| | %CV | 15.2 | 15.1 | 15.1 |

Conclusion: The controlled-release and immediate release formulations were well tolerated throughout the study, with no toxicities or serious adverse events observed. The results showed a robust, extended-release pattern for all three extended-release formulations (formulations not according to the invention) with mean plasma PAA concentrations exceeding those achieved with RAVICTI^{®} (glycerol phenylbutyrate) at all time points for at least 24 hours post dose. In addition, mean plasma PAGN concentrations and urinary PAGN excretion were greater for all three extended-release dosage forms than for RAVICTI^{®} at approximately the same molar PAA dose. It also demonstrated that urinary PAGN excretion for the immediate release formulation of L-ornithine phenylacetate was approximately twice as efficient as for RAVICTI^{®}.

## Claims

1. An immediate release oral pharmaceutical formulation for use in therapy, comprising L-ornithine phenylacetate at an oral dosage of about 2.0 g to about 9.0 g, and one or more pharmaceutically acceptable excipients or carriers, wherein the formulation is for administration as a single oral dose and provides an immediate release profile of L-ornithine phenylacetate upon oral administration.

2. An immediate release oral pharmaceutical formulation for use according to Claim 1, wherein the oral dosage of L-ornithine phenylacetate is about 2.0 g to about 4.0 g.

3. An immediate release oral pharmaceutical formulation for use according to any one of Claims 1 to 3, wherein the formulation is in two or more unit dosage forms.

4. An immediate release oral pharmaceutical formulation for use according to Claim 3. wherein the unit dosage form is a tablet, a capsule, a pill, pellets, free-flowing powder, or liquid.

5. An immediate release oral pharmaceutical formulation according to any one of Claims 1 to 4 for use in treating hyperammonemia.

6. An immediate release oral pharmaceutical formulation for use according to Claim 5, wherein the subject has acute liver failure or chronic liver disease.

7. An immediate release oral pharmaceutical formulation for use according to Claim 6, wherein the subject has liver cirrhosis or liver decompensation.

8. An immediate release oral pharmaceutical formulation for use according to Claim 5, wherein the subject has one or more conditions selected from the group consisting of hepatic encephalopathy, portal hypertension, and urea cycle disorder.

9. An immediate release oral pharmaceutical formulation for use according to any one of Claims 5 to 8, wherein said use comprises administering L-ornithine phenylacetate in an amount from about 5.0 g to about 30.0 g per day.

10. An immediate release oral pharmaceutical formulation for use according to any one of Claims 5 to 9, wherein the plasma Cmax of phenylacetic acid is from about 15 mg/L to about 120 mg/L.

11. An immediate release oral pharmaceutical formulation for use according to any one of Claims 5 to 9, wherein the plasma Cmax of phenylacetylglutamine is from about 15 mg/L to about 75 mg/L.

12. An immediate release oral pharmaceutical formulation for use according to any one of Claims 5 to 11, wherein said pharmaceutical formulation provides conversion of phenylacetate to phenylacetylglutamine over 24 hours of greater than about 80%.

13. An immediate release oral pharmaceutical formulation for use according to Claim 5, where said formulation comprises 3 to 5 unit dosage forms each comprising from about 1.0 g to about 1.5 g of L-ornithine phenylacetate.

14. An immediate release oral pharmaceutical formulation for use according to Claim 13, wherein the oral administration is repeated 2 or 3 times per day.

## Patentansprüche

1. Eine orale pharmazeutische Formulierung mit sofortiger Freisetzung zur Verwendung in der Therapie, umfassend L-Ornithinphenylacetat in einer oralen Dosierung von etwa 2.0 g bis etwa 9.0 g, und einem oder mehreren pharmazeutisch akzeptablen Hilfsstoffen oder Trägern, wobei die Formulierung zur Verabreichung als eine orale Einzeldosis bestimmt ist und bei oraler Verabreichung ein sofortiges Freisetzungsprofil von L-Ornithinphenylacetat liefert.

2. Eine orale pharmazeutische Formulierung mit sofortiger Freisetzung zur Verwendung nach Anspruch 1,
wobei die orale Dosierung von L-Ornithinphenylacetat etwa 2.0 g bis etwa 4.0 g beträgt.

3. Eine orale pharmazeutische Formulierung mit sofortiger Freisetzung zur Verwendung nach einem der Ansprüche 1 bis 3,
wobei die Formulierung in zwei oder mehr Einheitsdarreichungsformen vorliegt.

4. Eine orale pharmazeutische Formulierung mit sofortiger Freisetzung zur Verwendung nach Anspruch 3,
wobei die Einheitsdarreichungsform eine Tablette, eine Kapsel, eine Pille, Pellets, ein fließfähiges Pulver oder eine Flüssigkeit ist.

5. Eine orale pharmazeutische Formulierung mit sofortiger Freisetzung nach einem der Ansprüche 1 bis 4 zur Verwendung bei der Behandlung von Hyperammonämie.

6. Eine orale pharmazeutische Formulierung mit sofortiger Freisetzung zur Verwendung nach Anspruch 5, wobei das Subjekt akutes Leberversagen oder eine chronische Lebererkrankung aufweist.

7. Eine orale pharmazeutische Formulierung mit sofortiger Freisetzung zur Verwendung nach Anspruch 6, wobei das Subjekt Leberzirrhose oder eine Leberdekompensation aufweist.

8. Eine orale pharmazeutische Formulierung mit sofortiger Freisetzung zur Verwendung nach Anspruch 5, wobei das Subjekt einen oder mehrere Zustände aufweist, ausgewählt aus der Gruppe bestehend aus hepatischer Enzephalopathie, portaler Hypertension und Harnstoffzyklusstörungen.

9. Eine orale pharmazeutische Formulierung mit sofortiger Freisetzung zur Verwendung nach einem der Ansprüche 5 bis 8, wobei die Verwendung die Verabreichung von L-Ornithinphenylacetat in einer Menge von etwa 5.0 g bis etwa 30.0 g pro Tag umfasst.

10. Eine orale pharmazeutische Formulierung mit sofortiger Freisetzung zur Verwendung nach einem der Ansprüche 5 bis 9, wobei der Plasma-Cmax von Phenylessigsäure etwa 15 mg/L bis etwa 120 mg/L beträgt.

11. Eine orale pharmazeutische Formulierung mit sofortiger Freisetzung zur Verwendung nach einem der Ansprüche 5 bis 9, wobei der Plasma-Cmax von Phenylacetylglutamin etwa 15 mg/L bis etwa 75 mg/L beträgt.

12. Eine orale pharmazeutische Formulierung mit sofortiger Freisetzung zur Verwendung nach einem der Ansprüche 5 bis 11, wobei diese pharmazeutische Formulierung eine Umwandlung von Phenylacetat in Phenylacetylglutamin über 24 Stunden von mehr als etwa 80 % bewirkt.

13. Eine orale pharmazeutische Formulierung mit sofortiger Freisetzung zur Verwendung nach Anspruch 5, wobei diese Formulierung 3 bis 5 Einheitsdarreichungsformen umfasst, die jeweils etwa 1.0 g bis etwa 1.5 g L-Ornithinphenylacetat umfasst.

14. Eine orale pharmazeutische Formulierung mit sofortiger Freisetzung zur Verwendung nach Anspruch 13, wobei die orale Verabreichung 2 oder 3 mal pro Tag wiederholt wird.

## Revendications

1. Formulation pharmaceutique orale à libération immédiate destinée à être utilisée en thérapie, comprenant du pénylacétate de L-ornithine à une dose orale d'environ 2,0 g à environ 9,0 g, et un ou plusieurs excipients ou vecteurs pharmaceutiquement acceptables, où la formulation est destinée à être administrée sous forme d'une seule dose orale et procure un profil de libération immédiate de phénylacétate de L-ornithine lors de l'administration orale.

2. Formulation pharmaceutique orale à libération immédiate destinée à être utilisée selon la revendication 1,
où la dose orale de phénylacétate de L-ornithine est d'environ 2,0 g à environ 4,0 g.

3. Formulation pharmaceutique orale à libération immédiate destinée à être utilisée selon l'une quelconque des revendications 1 à 3, où la formulation est dans deux formes posologiques unitaires ou plus.

4. Formulation pharmaceutique orale à libération immédiate destinée à être utilisée selon la revendication 3,
où la forme posologique unitaire est un comprimé, une capsule, une pilule, des pastilles, une poudre fluide ou un liquide.

5. Formulation pharmaceutique orale à libération immédiate selon l'une quelconque des revendications 1 à 4 destinée à être utilisée dans le traitement de l'hyperammoniémie.

6. Formulation pharmaceutique orale à libération immédiate destinée à être utilisée selon la revendication 5, où le sujet a une insuffisance hépatique aiguë ou une maladie hépatique chronique.

7. Formulation pharmaceutique orale à libération immédiate destinée à être utilisée selon la revendication 6, où le sujet a une cirrhose du foie ou une décompensation du foie.

8. Formulation pharmaceutique orale à libération immédiate destinée à être utilisée selon la revendication 5, où le sujet a une ou plusieurs affections choisies dans le groupe consistant en l'encéphalopathie hépatique, l'hypertension portale et un trouble du cycle de l'urée.

9. Formulation pharmaceutique orale à libération immédiate destinée à être utilisée selon l'une quelconque des revendications 5 à 8, où ladite utilisation comprend l'administration de phénylacétate de L-ornithine en une quantité d'environ 5,0 g à environ 30,0 g par jour.

10. Formulation pharmaceutique orale à libération immédiate destinée à être utilisée selon l'une quelconque des revendications 5 à 9, où la Cmax plasmatique de l'acide phénylacétique est d'environ 15 mg/L à environ 120mg/L.

11. Formulation pharmaceutique orale à libération immédiate destinée à être utilisée selon l'une quelconque des revendications 5 à 9, où la Cmax plasmatique de la phénylacétylglutamine est d'environ 15 mg/L à environ 75mg/L.

12. Formulation pharmaceutique orale à libération immédiate destinée à être utilisée selon l'une quelconque des revendications 5 à 11, où ladite formulation pharmaceutique procure une conversion du phénylacétate en phénylacétylglutamine en 24 heures supérieure à environ 80 %.

13. Formulation pharmaceutique orale à libération immédiate destinée à être utilisée selon la revendication 13, où ladite formulation comprend 3 à 5 formes posologiques unitaires comprenant chacune d'environ 1,0 g à environ 1,5 g de phénylacétate de L-ornithine.

14. Formulation pharmaceutique orale à libération immédiate destinée à être utilisée selon la revendication 13, où l'administration orale est répétée 2 ou 3 fois par jour.
